(19) Europäisches Patentamt
European Patent Office
Office européen des brevets

(11) Publication number: 0 288 563 B1

# (12) EUROPEAN PATENT SPECIFICATION

(45) Date of publication of patent specification: 11.05.94

(51) Int. Cl.5: C07D 211/14, C07D 211/18, C07D 211/22, C07D 211/32, C07D 211/70, C07D 295/18, C07D 401/06, C07D 405/04, C07D 409/04, A61K 31/445, A61K 31/47

(21) Application number: 86905925.3

(22) Date of filing: 30.09.86

(86) International application number: PCT/JP86/00502

(87) International publication number: WO 88/02365 (07.04.88 88/08)

The file contains technical information submitted after the application was filed and not included in this specification

## (54) CYCLIC AMINE DERIVATIVES.

(43) Date of publication of application: 02.11.88 Bulletin 88/44

(45) Publication of the grant of the patent: 11.05.94 Bulletin 94/19

(84) Designated Contracting States: AT BE CH DE FR GB IT LI LU NL SE

(56) References cited:

EP-A- 0 109 317
EP-A- 0 202 164
DE-A- 2 027 446
FR-A- 2 163 358
FR-M- 7 431
US-A- 3 576 810
US-A- 3 878 217
US-A- 3 946 022
US-A- 4 235 914
EP-A- 0 193 875
EP-A- 0 325 268
FR-A- 2 105 119
FR-A- 2 227 868
GB-A- 2 041 918
US-A- 3 806 526
US-A- 3 922 276
US-A- 3 954 871
US-B- 407 737

(73) Proprietor: Eisai Co., Ltd.
6-10, Koishikawa 4-chome
Bunkyo-ku
Tokyo 112(JP)

(72) Inventor: SUGIMOTO, Hachiro
3073-13, Kashiwadamachi
Ushiku-shi Ibaraki 300-12(JP)
Inventor: NAKAMURA, Takaharu
4-9, Tsukushino 2-chome
Abiko-shi Chiba 270-11(JP)
Inventor: KARIBE, Norio
Eisai Shizanryo, 19-13
Kasuga 4-chome
Yatabemachi Tsukuba-gun Ibaraki 305(JP)
Inventor: SAITO, Isao
670-63, Shimohirooka
Sakuramura
Niihari-gun Ibaraki 305(JP)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

CHEMICAL ABSTRACTS, vol. 106, no. 25, 22nd June 1987, page 645, abstract no. 213767z, Columbus, Ohio, US

CHEMICAL ABSTRACTS, vol. 82, no. 25, 23rd June 1975, page 545, abstract no. 170925r, Columbus, Ohio, US

JOURNAL OF MEDICINAL CHEMISTRY, vol. 13, no. 1, January 1970, pages 1-6, The American Chemical Society; R.L. DUNCAN et al.: "Aroylpiperidines and pyrrolidines. A new class of potent central nervous system depressants"

Inventor: **HIGURASHI, Kunizo**
**4-3, Midori 2-chome**
**Sumida-ku, Tokyo 130(JP)**
Inventor: **YONAGA, Masahiro**
**Yuhara mansion 203, 110-8**
**Arakawaoki**
**Tsuchiura-shi Ibaraki 300-11(JP)**
Inventor: **KANEKO, Takeru**
**17-6-1, Matsushiro 2-chome**
**Yatabemachi, Tsukuba-gun Ibaraki 305(JP)**
Inventor: **NAKAZAWA, Takahiro**
**531-1-810, Ohaza Miyawada**
**Fujishiromachi**
**Kitasouma-gun Ibaraki 300-15(JP)**
Inventor: **UENO, Masataka**
**Parkside Doho, 17-10**
**Ninomiya 2-chome**
**Yatabemachi, Tsukuba-gun Ibaraki 305(JP)**
Inventor: **YAMATSU, Kiyomi**
**23-7, Imaizumidai 7-chome**
**Kamakura-shi Kanagawa 247(JP)**

(74) Representative: **Lehn, Werner, Dipl.-Ing. et al**
**Hoffmann, Eitle & Partner,**
**Patentanwälte,**
**Postfach 81 04 20**
**D-81904 München (DE)**

## Description

The present invention relates to cyclic amine derivatives having excellent medicinal activities.

Prior Art:

Various medicines for cerebral vascular disorders have been proposed. For example, cerebral vasodilator drugs and cerebral metabolism activators have been used. However, no drug which is drastically effective has been proposed as yet. At present, there is no drug effective particularly for cerebral vascular dementia and intellectual function disorders among the symptoms due to cerebral vascular disorders.

Object of the Invention:

After intensive investigations made for the purpose of finding a new compound effective for the treatment of various symptoms due to cerebral vascular disorders, particularly mental symptoms, over a long time under the above-mentioned circumstances, the inventors have found quite effective compounds. The present invention has been completed on the basis of this finding.

Therefore, an object of the present invention is to provide cyclic amine derivatives and pharmacologically acceptable salts thereof which are effective for the treatment of cerebral vascular disorders such as cerebral stroke, apoplexy, infarction and arteriosclerosis and mental symptoms due to multiple infarct dementia. Another object of the invention is to provide a process for producing said compounds or pharmacologically acceptable salts thereof. Still another object of the invention is to provide medicines containing said compound or pharmacologically acceptable salt thereof as the active ingredient.

Construction and Effect of the Invention:

The intended compounds of the present invention are cyclic amine derivatives of the general formula (I) or pharmacologically acceptable salts thereof:

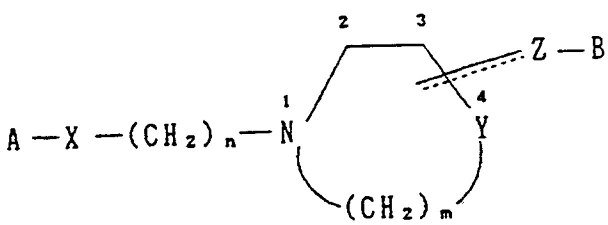

( I )

wherein A represents a substituted or unsubstituted phenyl, pyridyl or thienyl group, substituted or unsubstituted naphthyl, tetralyl, quinolyl, benzofuranyl, quinazolyl or benzothienyl group or a group of the formula:

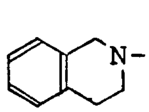 or 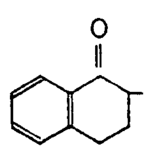 ,

X represents a group of the formula:
$-CH_2-$,

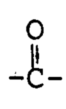, 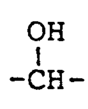, 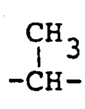 or 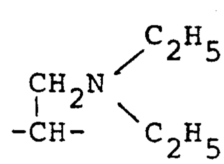 ,

n represents an integer of 0 to 4,
m represents an integer of 1 to 3,
Y represents a carbon or nitrogen atom,
Z represents a group of the formula: $-CH_2-$,

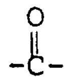 , 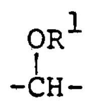

in which $R^1$ is a hydrogen atom or a lower alkyl, acyl, arylalkyl or heteroarylalkyl group,

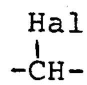

in which Hal is a halogen atom, =CH-,

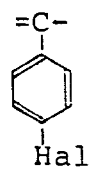

in which Hal is a halogen atom,

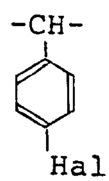

in which Hal is a halogen atom or

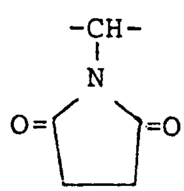 ,

the symbol

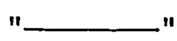

between the ring and Z represents a single or double bond,

the group of the formula:

"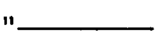 Z-B"

is bonded with the ring in the above formula at the 3- or 4-position, and

B represents a phenyl or naphthyl group which may be substituted with one or two substituents which may be the same or different and which are selected from the group consisting of halogens, lower alkyl groups and lower alkoxy groups.

The lower alkyl groups in the above-mentioned definitions of $R^1$ and B include, for example, straight-chain or branched alkyl groups having 1 to 6 carbon atoms such as methyl, ethyl, n-propyl, n-butyl, isopropyl, isobutyl, 1-methylpropyl, tert-butyl, n-pentyl, 1-ethylpropyl, isoamyl and n-hexyl groups. Among them, methyl and ethyl groups are the most preferred.

The lower alkoxy groups in the above-mentioned definition of B are those derived from the above-mentioned lower alkyl groups. Preferred examples of them include methoxy, ethoxy, propoxy, isopropoxy, butoxy and isobutoxy groups.

The substituents of the "substituted or unsubstituted phenyl group" and "substituted or unsubstituted naphthyl group" in the definition of A include, for example, the above-defined lower alkyl and alkoxy groups, hydroxyl group, halogen atoms such as fluorine, bromine, iodine and chlorine, phenyl group and heterocyclic groups having nitrogen atom(s) as the hetero atom such as imidazolyl, pyridyl and pyrazolyl groups. Said compounds may have one to three of these substituents. When the compound have two or more of these substituents, they may be the same or different.

The phenyl group may have a methylenedioxy or ethylenedioxy group bonded with two different carbon atoms constituting the phenyl ring in addition to the above-mentioned substituents. Further, the substituted phenyl group include also a group of the formula:

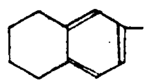

The acyl groups in the definition of $R^1$ include organic acid residues such as saturated aliphatic, unsaturated aliphatic, carbocyclic and heterocyclic carboxylic acid residues. Examples of them include lower alkanoyl groups such as formyl, acetyl, propionyl, butyryl, isobutyryl, valeryl, isovaleryl and pivaloyl groups, aroyl groups such as benzoyl, toluoyl and naphthoyl groups and heteroaroyl groups such as furoyl, nicotinoyl and isonicotinoyl groups.

The arylalkyl groups in the definition of $R^1$ include, for example, those derived from substituted or unsubstituted phenyl and naphthyl groups. Typical examples of them include benzyl and phenethyl groups. The substituents in the above definition include, for example, the above-defined lower alkyl and lower alkoxy groups, hydroxyl group and halogen atoms such as fluorine, bromine, iodine and chlorine atoms.

Typical examples of the heteroarylalkyl groups include pyridylalkyl groups such as picolyl group.

The halogen atoms include fluorine, chlorine, bromine and iodine atoms.

The phamacologically acceptable salts are ordinary non-toxic salts, for example, inorganic acid salts such as hydrochlorides, hydrobromides, sulfates and phosphates; organic acid salts such as acetates, maleates, tartrates, methanesulfonates, benzenesulfonates and toluenesulfonates; and amino acid salts such as arginine salts, aspartates and glutamates.

Production processes

The compounds of the present invention can be produced by various processes. A typical example of these processes comprises:

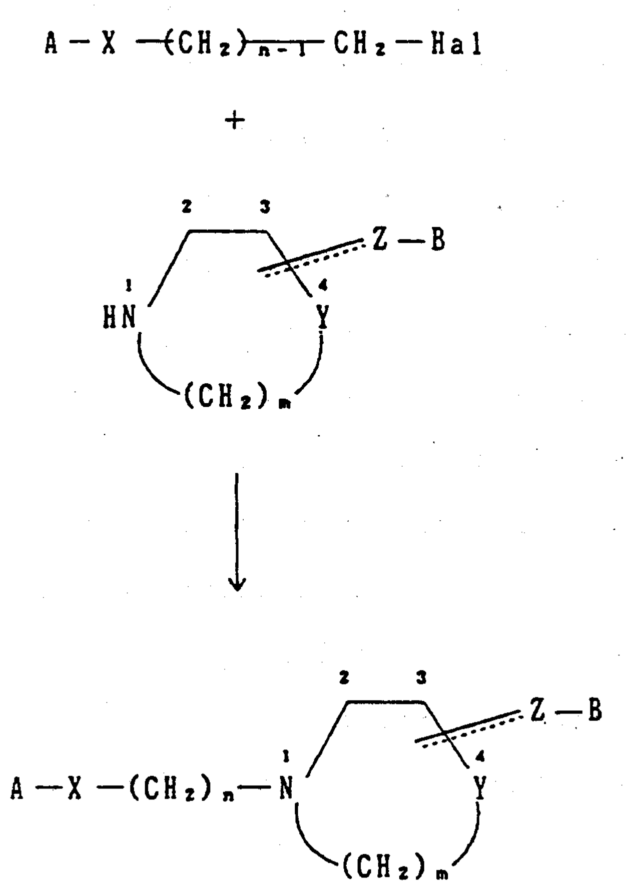

( II )

( III )

( I )

wherein Hal represents a halogen atom and A, X, Y, Z, B, n, m and

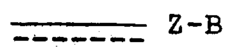

are as defined above.

Namely, a halide of the general formula (II) is reacted with a compound of the general formula (III) to obtain an intended compound of the general formula (I).

The dehydrohalogenation reaction is carried out by heating in an ordinary manner without using any solvent or in an organic solvent inert to the reaction which is selected from the group consisting of alcoholic solvents such as methanol, ethanol and butanol, benzene, toluene, xylene, tetrahydrofuran, chloroform, carbon tetrachloride and dimethylformamide. Preferred results are obtained when the reaction is carried out in the presence of an inorganic salt such as sodium hydrogencarbonate, potassium carbonate, sodium carbonate or sodium hydroxide or an organic base such as triethylamine, pyridine, pyrimidine or diethylaniline.

It is apparent from the pharmacological experiments described below that the compounds of the present invention have excellent pharmacological effects on the central nervous system, particularly a remarkable reparative effect on ischemic cerebral vascular disorders. Therefore, these compounds are useful for relieving, remedying or preventing mental disorders due to the cerebral vascular disorders such as cerebral stroke, apoplexy, infarction, arteriosclerosis and dementias, e.g. multiple infarct dementia.

It has been found in toxicity tests effected by using rats that the compounds of the present invention have a high safety and, therefore, the invention is highly valuable also in this regard.

According to the toxicity tests of typical compounds of the present invention (see Examples 1 to 12 given below), $LD_{50}$ of them was 2,000 to 4,000 mg/kg (oral administration to rats).

The compounds of the present invention used as the medicine are given either orally or parenterally. The dose of said compounds is not particularly limited, since it varies depending on the symptoms; age, sex, body weight and sensitivity of the patient; period and intervals of the administration; properties, composition and kind of the medicinal preparation; and varieties of active ingredients. Usually, about 0.1 to

300 mg/day, preferably about 1 to 100 mg/day of the compound is administered 1 to 4 times a day.

The compounds of the present invention are used in the form of a medicinal preparation such as an injection, suppository, sublingual tablet, tablet or capsule.

In the preparation of the injection, a pH adjustor, buffer, suspending agent, solubilizer, stabilizer, isotonizer, preservative, etc. are added to the active ingredient to form an intravenous, subcutaneous or intramuscular injection by an ordinary method. If necessary, the injection can be freeze-dried by an ordinary method.

Examples of the suspending agents include methylcellulose, Polysorvate 80, hydroxyethylcellulose, acacia, tragacanth gum powder, sodium carboxymethylcellulose and polyoxyethylenesorbitan monolaurate.

Examples of the solubilizers include polyoxyethylene hardened castor oil, Polysorbate 80, nicotinamide, polyoxyethylene sorbitan monolaurate, macrogol and ethyl esters of castor oil fatty acids.

Examples of the stabilizers include sodium sulfite, sodium metasulfite and ether. Examples of the preservatives include methyl hydroxybenzoate, ethyl hydroxybenzoate, sorbic acid, phenol, cresol and chlorocresol.

[Examples]

Typical examples of the compounds of the present invention will be shown below for facilitating the understanding of the present invention, which by no means limit the scope of the invention.

Example 1

2-{2-[4-(p-Fluorobenzyl)piperidinyl]ethyl}naphthalene hydrochloride:

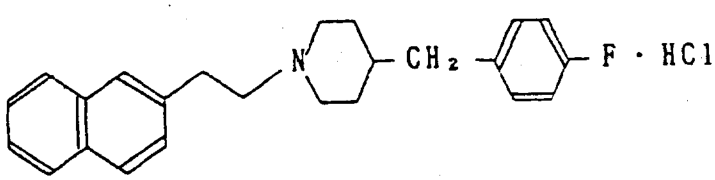

1.05 g of 1-chloro-2-(2-naphthyl)ethane, 1.09 g of 4-(p-fluorobenzyl)piperidine, 0.2 g of potassium iodide and 1.4 g of sodium hydrogencarbonate were refluxed in n-butanol solvent for 5 h. Then, the solvent was filtered out and 100 mℓ of chloroform was added to the residue. The mixture was washed with water and dried over magnesium sulfate. The oily product thus obtained was purified according to silica gel column chromatography and converted into its hydrochloride by an ordinary method.

Yield: 0.45 g

Melting point: 244 °C

| Elementary analysis for $C_{24}H_{26}NF \cdot HCl$: | | | |
|---|---|---|---|
| | C | H | N |
| calculated (%): | 75.08 | 7.09 | 3.65 |
| found (%): | 75.30 | 7.32 | 7.34 |

Example 2

<u>2-(4-Benzylpiperidinyl)-2'-acetonaphthone hydrochloride</u>:

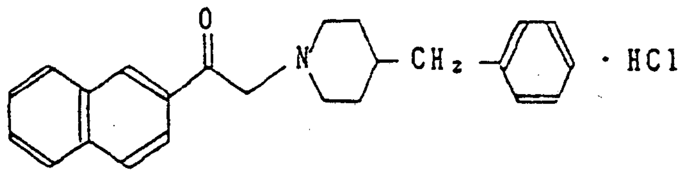

5 g of 2-bromo-2'-acetonaphthone, 3.5 g of 4-benzylpiperidine, 0.2 g of potassium iodine and 5 g of sodium hydrogencarbonate were refluxed in butanol solvent for 4 h. After completion of the reaction, the product was treated by an ordinary process. The oily product thus obtained was purified according to silica gel column chromatography and converted into its hydrochloride, which was then recrystallized from chloroform and ethanol.
Yield: 2.1 g
Melting point: 233 to 235 ° C

| Elementary analysis for $C_{24}H_{25}NO \cdot HCl$: | | | |
|---|---|---|---|
| | C | H | N |
| calculated (%) | 75.87 | 6.90 | 3.69 |
| found (%) | 75.67 | 6.71 | 3.49 |

Example 3

<u>2-[4-Bis(4-fluorophenyl)methylene-1-piperidinyl]-2'-acetonaphthone hydrochloride</u>:

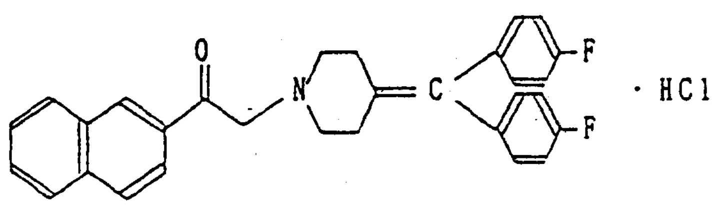

850 mg of 4-bis(4-fluorophenyl)methylenepiperidine, 700 mg of 2-bromo-2'-acetonaphthone, 20 mg of potassium iodide and 760 mg of sodium hydrogencarbonate were refluxed in n-butanol solvent for 3.5 h. After completion of the reaction, the product was treated by an ordinary process. The obtained oily product was purified according to silica gel column chromatography and converted into its hydrochloride to obtain 510 mg of the intended product.
Melting point: 214 to 217 ° C

| Elementary analysis for $C_{30}H_{25}NOF_2 \cdot HCl$ | | | |
|---|---|---|---|
| | C | H | N |
| calculated (%): | 73.54 | 5.35 | 2.86 |
| found (%) : | 73.54 | 5.46 | 3.03 |

Example 4

4-(1-Naphthonyl)piperidinyl-3',4'-dimethylacetophenone hydrochloride:

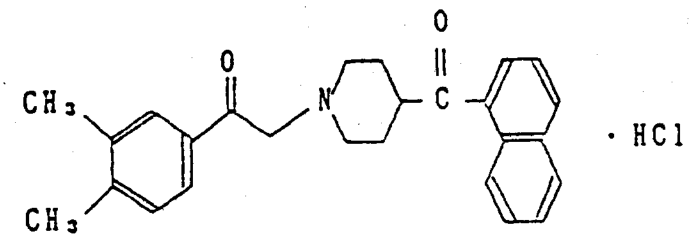

1.9 g of 2-bromo-3',4'-dimethylacetophenone, 2.0 g of 4-(1-naphthonyl)piperidine, 0.1 g of potassium iodide and 2.1 g of sodium hydrogencarbonate were refluxed in n-butanol solvent for 3 h. After completion of the reaction, the product was treated by an ordinary process. The obtained oily product was purified according to silica gel column chromatography and converted into its hydrochloride to obtain 1.0 g of the intended product.
Melting point: 92 to 96 °C (dec.)

| Elementary analysis for $C_{26}H_{27}NO_2 \cdot HCl$: | | | |
|---|---|---|---|
| | C | H | N |
| calculated (%) | 74.01 | 6.68 | 3.32 |
| found (%) | 73.79 | 6.69 | 3.01 |

Example 5

1-[3-(p-Fluorobenzoyl)piperidinyl]-2'-acetonaphthone hydrochloride:

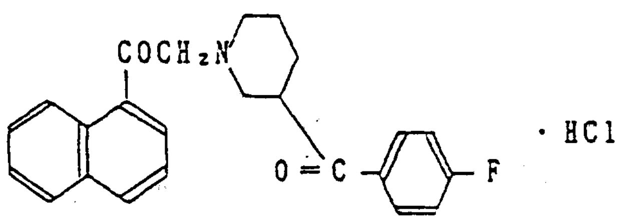

0.7 g of 1-bromo-2'-acetonaphthone, 0.7 g of 3-(p-fluorobenzoyl)piperidine hydrochloride, 0.05 g of potassium iodide and 0.7 g of sodium hydrogencarbonate were refluxed in n-butanol solvent for 2 h. After completion of the reaction, the product was treated by an ordinary process. The obtained oily product was purified according to silica gel column chromatography and converted into its hydrochloride.
Yield: 0.4 g
Melting point: 123 to 127 °C (dec.)

| Elementary analysis for $C_{24}H_{22}NO_2F \cdot HCl$: | | | |
|---|---|---|---|
| | C | H | N |
| calculated (%) | 69.98 | 5.63 | 3.40 |
| found (%) | 69.76 | 5.51 | 3.18 |

Example 6

2-[4-(α-Benzyloxy-p-fluorobenzyl)piperidinyl]-2'-acetonaphthone hydrochloride:

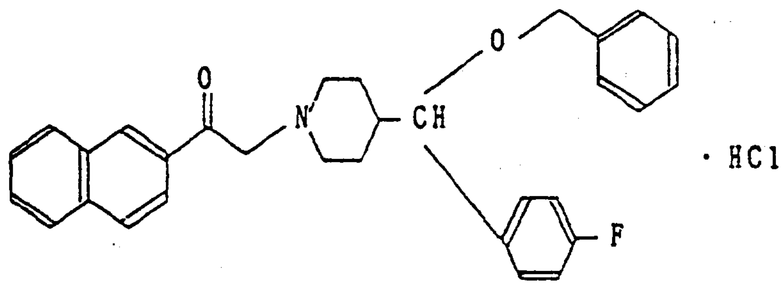

1.1 g of 2-bromo-2'-acetonaphthone, 1.2 g of 4-(α-benzyloxy-p-fluorobenzyl)piperidine and 4.5 g of sodium hydrogencarbonate were refluxed in ethanol solvent for 3.5 h. After completion of the reaction, the product was treated by an ordinary process. The oily product thus obtained was purified according to silica gel column chromatography and converted into its hydrochloride, which was recrystallized from ethyl acetate/methanol.
Yield: 0.6 g
Melting point: 115 to 120°C

| Elementary analysis for $C_{31}H_{30}NO_2F \cdot HCl$: | | | |
|---|---|---|---|
| | C | H | N |
| calculated (%) | 76.76 | 6.44 | 2.89 |
| found (%) | 76.59 | 6.21 | 2.68 |

Example 7

2-[4-(α-Acetoxy-p-fluorobenzyl)piperidinyl]-2'-acetonaphthone hydrochloride

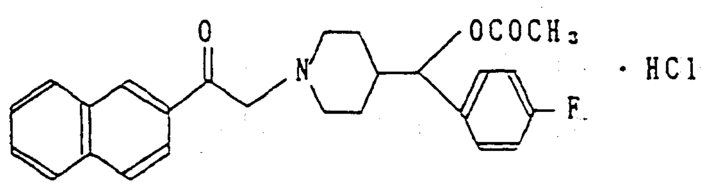

5.4 g of 2-bromo-2'-acetonaphthone, 4.6 g of 4-(α-hydroxy-p-fluorobenzyl)piperidine and 10 g of sodium hydrogencarbonate were refluxed in ethanol solvent for 2.5 h. After completion of the reaction, the product was treated by an ordinary process. The obtained oily product was purified according to silica gel column chromatography to obtain 5 g of 2-[4-(α-hydroxy-p-fluorobenzyl)piperidinyl]-2'-acetonaphthone, 1 g of this product was stirred together with 1.0 g of acetic anhydride and 0.1 g of dimethylaminopyridine in pyridine solvent at room temperature for 5 h. After completion of the reaction, the oily product was purified according to silica gel column chromatography and converted into its hydrochloride, which was recrystallized from ethyl acetate and methanol.
Yield: 1.0 g
Melting point: 148 to 152°C

| Elementary analysis for $C_{26}H_{26}NO_3F \cdot HCl$: | | | |
|---|---|---|---|
| | C | H | N |
| calculated (%) | 68.49 | 5.97 | 3.07 |
| found (%) | 68.24 | 5.88 | 3.12 |

Example 8

4-(4-p-Fluorobenzoyl)piperidinyl-6,7-dimethoxyisoquinoline hydrochloride

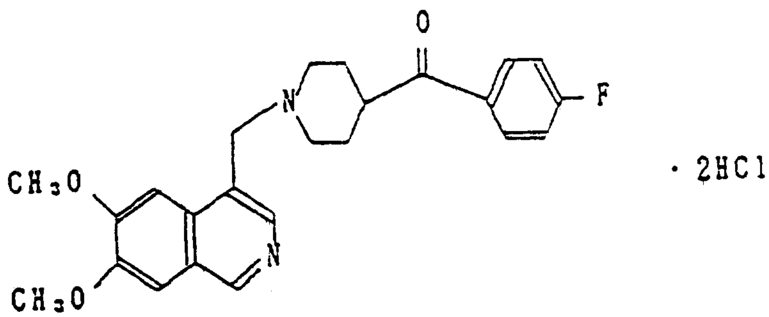

70 mg of 4-chloromethyl-6,7-dimethoxyisoquinoline was dissolved in 10 mℓ of dimethyl sulfoxide. 1 mℓ of triethylamine and 140 mg of 4-(p-fluorobenzoyl)piperidine were added to the solution and the mixture was heated to 80 °C for 1 h. The reaction mixture was dissolved in ethyl acetate, washed with water and dried over magnesium sulfate. The product was purified according to silica gel column chromatography and converted into its hydrochloride.
Yield: 80 mg
Melting point: 185 to 190 °C

| Elementary analysis for $C_{24}H_{25}N_2O_3F \cdot 2HCl$: | | | |
|---|---|---|---|
| | C | H | N |
| calculated (%) | 59.88 | 5.65 | 5.82 |
| found (%) | 59.78 | 5.61 | 5.80 |

Example 9

4-{2-[4-(p-Fluorobenzoyl)piperidinyl]ethyl}quinazoline hydrochloride

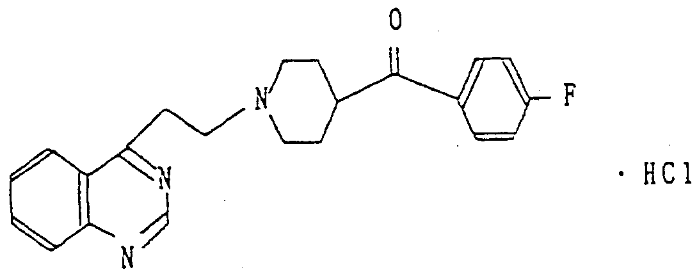

2 g of 4-methylquinazoline was dissolved in 20 mℓ of ethanol. 3.4 g of 4-(p-fluorobenzoyl)piperidine hydrochloride and 1.9 mℓ of 37% formalin were added to the solution and the mixture was stirred at room temperature for three days. A white precipitate was recovered by filtration and washed with ethanol to obtain the intended product.
Yield: 4.4 g
Melting point: 135 to 140 °C

| Elementary analysis for $C_{22}H_{22}N_3OF \cdot HCl$: | | | |
|---|---|---|---|
| | C | H | N |
| calculated (%) | 66.08 | 5.79 | 10.51 |
| found (%) | 66.02 | 5.65 | 10.44 |

Example 10

1-(2-Naphthyl)-1-[4-(p-fluorobenzoyl)piperidinyl]-2-diethylaminoethane hydrochloride:

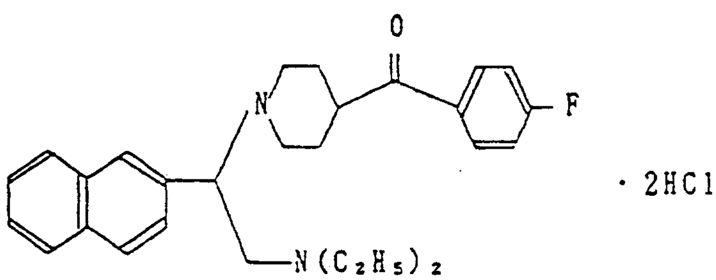

1.4 g of 1-(2-naphthyl)-2-diethylaminoethanol was dissolved in 20 mℓ of dichloromethane. 2.4 mℓ of triethylamine and 0.9 mℓ of methanesulfonyl chloride were added to the solution under cooling with ice and the mixture was stirred at room temperature for 4.5 h. A solution of 1.2 g of 4-(p-fluorobenzoyl)piperidine in 25 mℓ of dioxane was added to the reaction mixture and the obtained mixture was refluxed for 2 h. After completion of the reaction, the product was purified according to silica gel column chromatography and then converted into its hydrochloride.
Yield: 1.9 g
Melting point: 140 to 145 °C

| Elementary analysis for $C_{28}H_{33}N_2OF \cdot 2HCl$: | | | |
|---|---|---|---|
| | C | H | N |
| calculated (%)<br>found (%) | 66.52<br>66.57 | 6.97<br>6.81 | 5.54<br>5.38 |

Example 11

2-[4-(α-Succinimido-p-fluorobenzyl)piperidinyl]-2'-acetonephthone hydrochloride

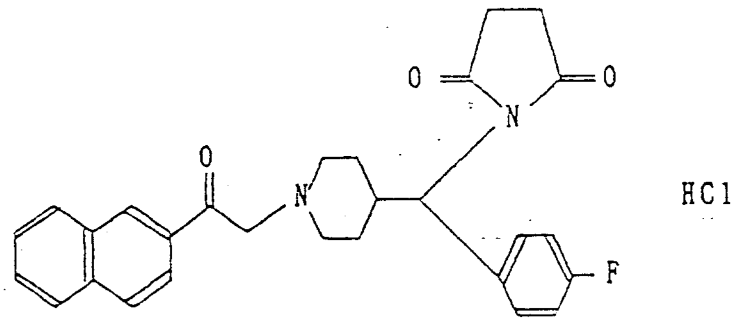

470 mg of 4-(α-succinimido-p-fluorobenzyl)piperidine was dissolved in 40 mℓ of ethanol. 410 mg of 2-bromo-2'-acetonaphthone and 420 mg of sodium hydrogencarbonate were added to the solution and the mixture was refluxed for 30 min. After completion of the reaction, the prodcut was treated by an ordinary process. The obtained product was purified according to silica gel column chromatography and converted into its hydrochloride.
Yield: 400 mg
Melting point: 233 to 237 °C

| Elementary analysis for $C_{28}H_{27}N_2O_3F \cdot HCl$: | | | |
|---|---|---|---|
| | C | H | N |
| calculated (%)<br>found (%) | 67.94<br>68.13 | 5.70<br>5.56 | 5.66<br>5.47 |

Example 12

2-[4-p-Fluorobenzoyl)piperidinyl]-2'-acetonaphthone hydrochloride

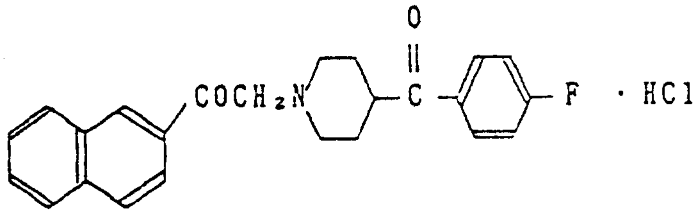

49.7 g of 2-bromo-2'-acetonaphthone, 49.9 g of 4-(p-fluorobenzoyl)piperidine hydrochloride, 0.5 g of potassium iodide and 50.4 g of sodium hydrogencarbonate were added to 500 mℓ of ethanol and the

mixture was refluxed for 2 h. The solvent was distilled off and chloroform was added to the residue. The mixture was washed with water and dried. Chloroform was distilled off and the residue was purified according to silica gel column chromatography to obtain 58.9 g of the crystalline intended product, which was converted into its hydrochloride and recrystallized by an ordinary process to obtain the intended hydrochloride.
Melting point: 247 to 248 °C (dec.)

| Elementary analysis for $C_{24}H_{22}NO_2F \cdot HCl$: | | | |
|---|---|---|---|
| | C | H | N |
| calculated (%) | 69.98 | 5.63 | 3.40 |
| found (%) | 69.81 | 5.51 | 3.36 |

Examples 13 to 95

Compounds shown in Table 1 were prepared in the same manner as in Examples 1 to 12.

Table 1

| Example No. | Structural formula | Melting point (°C) | Chemical formula | Elementary analysis (%) calculated/found C | H | N |
|---|---|---|---|---|---|---|
| 13 | 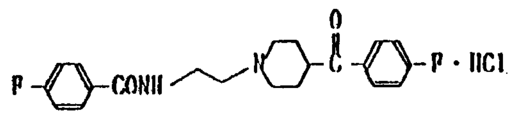 | 234~235 (dec.) | $C_{21}H_{23}N_2O_2F_2 \cdot HCl$ | 61.68<br>61.49 | 5.92<br>5.85 | 6.85<br>6.77 |
| 14 | 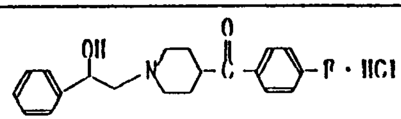 | 216~218 (dec.) | $C_{20}H_{22}NO_2F \cdot HCl$ | 66.02<br>66.16 | 6.37<br>6.39 | 3.85<br>3.77 |
| 15 | 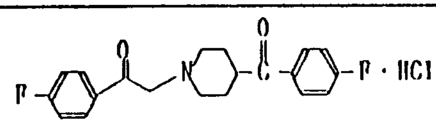 | 228~229 (dec.) | $C_{20}H_{19}NO_2F_2 \cdot HCl$ | 63.24<br>63.11 | 5.31<br>5.37 | 3.69<br>3.58 |
| 16 | 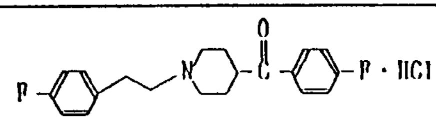 | 223~224 (dec.) | $C_{20}H_{21}NOF_2 \cdot HCl$ | 65.66<br>65.39 | 6.06<br>6.12 | 3.83<br>3.81 |
| 17 | 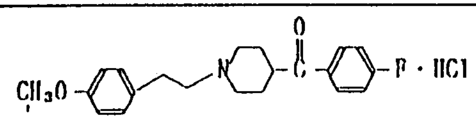 | 225~226 (dec.) | $C_{21}H_{24}NO_2 \cdot HCl$ | 70.28<br>69.97 | 7.02<br>7.13 | 3.90<br>3.88 |
| 18 | 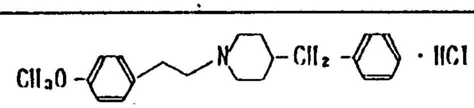 | 201~202 | $C_{21}H_{27}NO \cdot HCl$ | 72.92<br>72.76 | 8.16<br>8.23 | 4.05<br>4.11 |

Table 1 (cont'd)

| Example No. | Structural formula | Melting point (°C) | Chemical formula | Elementary analysis (%) calculated/found | | |
|---|---|---|---|---|---|---|
| | | | | C | H | N |
| 19 | 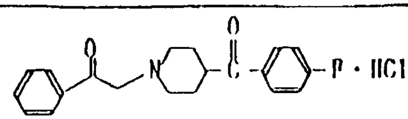 | 233~235 (dec.) | $C_{20}H_{20}NO_2F \cdot HCl$ | 66.38<br>66.27 | 5.85<br>5.82 | 3.87<br>3.85 |
| 20 | 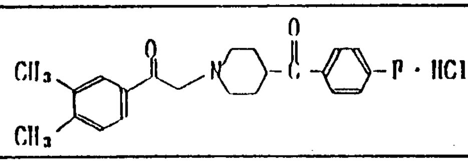 | 244~245 | $C_{22}H_{24}NO_2F \cdot HCl$ | 67.77<br>67.80 | 6.46<br>6.47 | 3.59<br>3.57 |
| 21 | 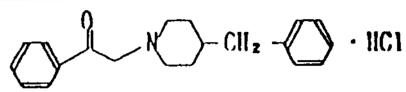 | 211~211.5 | $C_{20}H_{23}NO \cdot HCl$ | 72.82<br>72.19 | 7.33<br>7.13 | 4.25<br>4.12 |
| 22 | 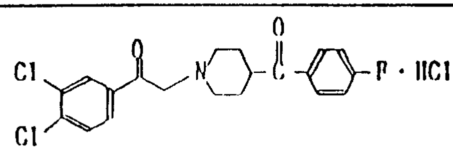 | 222~223 (dec.) | $C_{20}H_{18}NO_2FCl_2 \cdot 2HCl$ | 55.75<br>55.71 | 4.44<br>4.50 | 3.25<br>3.26 |
| 23 | 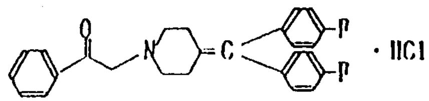 | 235~236 | $C_{26}H_{23}NOF_2 \cdot HCl$ | 70.98<br>70.59 | 5.50<br>5.63 | 3.18<br>3.34 |

**Table 1 (cont'd)**

| Example No. | Structural formula | Melting point (°C) | Chemical formula | Elementary analysis (%) calculated/found | | |
|---|---|---|---|---|---|---|
| | | | | C | H | N |
| 24 | 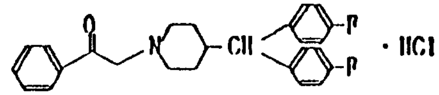 | 143~146 | $C_{26}H_{25}NOF_2 \cdot HCl$ | 70.66<br>70.43 | 5.93<br>5.91 | 3.17<br>3.24 |
| 25 | 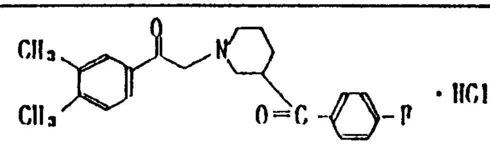 | 65~69 | $C_{22}H_{24}NO_4F \cdot HCl$ | 62.63<br>62.48 | 5.97<br>5.70 | 3.39<br>3.11 |
| 26 | 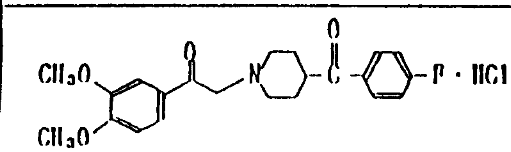 | 234~236 (dec.) | $C_{22}H_{24}NO_4F \cdot HCl$ | 62.63<br>62.57 | 5.97<br>5.96 | 3.32<br>3.15 |
| 27 | 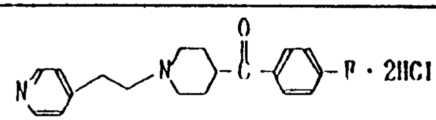 | 223~226 (dec.) | $C_{19}H_{21}N_2OF \cdot 2HCl$ | 59.23<br>59.18 | 6.02<br>6.11 | 7.27<br>7.02 |
| 28 | 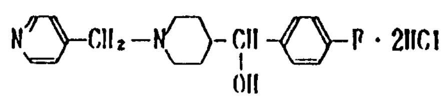 | 155~160 (dec.) | $C_{18}H_{21}N_2OF \cdot 2HCl$ | 57.91<br>57.80 | 6.21<br>6.12 | 7.50<br>7.20 |

Table 1 (cont'd)

| Example No. | Structural formula | Melting point (°C) | Chemical formula | Elementary analysis (%) calculated/found | | |
|---|---|---|---|---|---|---|
| | | | | C | H | N |
| 29 | 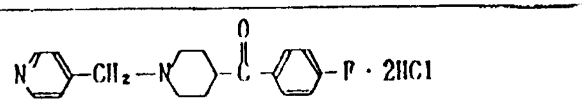 | 220~225 (dec.) | $C_{18}H_{19}N_2OF \cdot 2HCl$ | 58.23<br>58.25 | 5.70<br>5.71 | 7.55<br>7.43 |
| 30 | 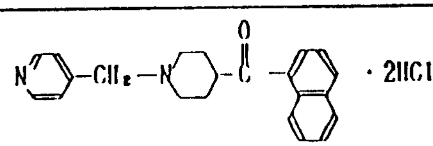 | 121~125 (dec.) | $C_{22}H_{22}N_2O \cdot HCl$ | 63.01<br>62.91 | 5.77<br>5.73 | 6.68<br>6.59 |
| 31 | 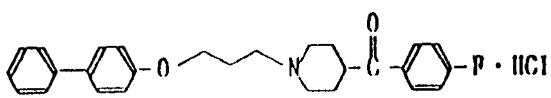 | 238~240 | $C_{27}H_{28}NO_2F \cdot HCl$ | 71.42<br>71.13 | 6.44<br>6.29 | 3.09<br>3.10 |
| 32 | 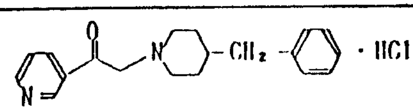 | 173~174 | $C_{19}H_{22}N_2O \cdot HCl$ | 68.98<br>68.75 | 7.01<br>6.89 | 8.47<br>8.26 |
| 33 | 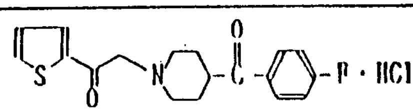 | 243~244 | $C_{18}H_{18}NO_2F \cdot HCl$ | 58.77<br>58.61 | 5.21<br>5.51 | 3.81<br>3.91 |

Table 1 (cont'd)

| Example No. | Structural formula | Melting point (°C) | Chemical formula | Elementary analysis (%) calculated/found | | |
|---|---|---|---|---|---|---|
| | | | | C | H | N |
| 34 | 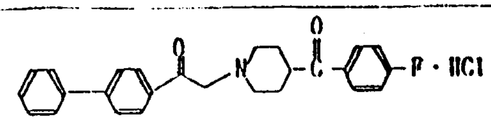 | 253~254 (dec.) | $C_{26}H_{24}NO_2F \cdot HCl$ | 71.31<br>71.51 | 5.75<br>6.03 | 3.20<br>3.25 |
| 35 | 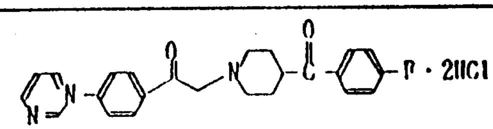 | 269~270 (dec.) | $C_{23}H_{22}N_3O_2F \cdot 2HCl$ | 59.36<br>59.23 | 5.41<br>5.40 | 9.03<br>9.11 |
| 36 | 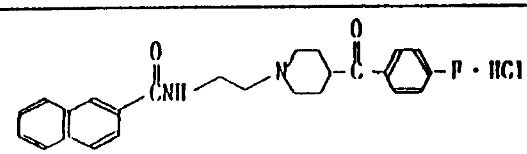 | 182~184 (dec.) | $C_{25}H_{25}N_2O_2F \cdot HCl$ | 68.10<br>68.31 | 5.94<br>5.96 | 6.35<br>6.22 |
| 37 | 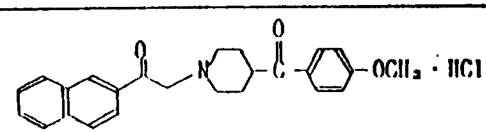 | 232~234 (dec.) | $C_{25}H_{25}NO_3 \cdot HCl$ | 70.83<br>70.76 | 6.18<br>6.09 | 3.30<br>3.21 |
| 38 | 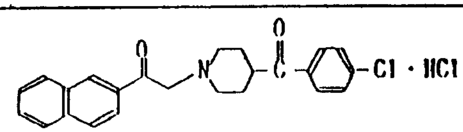 | 242~244 (dec.) | $C_{24}H_{22}NO_2Cl \cdot HCl$ | 67.30<br>67.22 | 5.41<br>5.35 | 3.27<br>3.31 |

Table 1 (cont'd)

| Example No. | Structural formula | Melting point (°C) | Chemical formula | Elementary analysis (%) calculated/found | | |
|---|---|---|---|---|---|---|
| | | | | C | H | N |
| 39 | 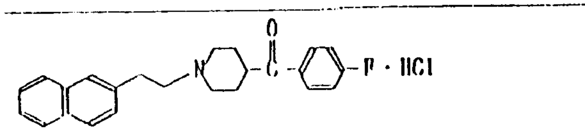 | 253~255 (dec.) | $C_{24}H_{24}NOF \cdot HCl$ | 72.44<br>72.40 | 6.33<br>6.38 | 3.52<br>3.49 |
| 40 | 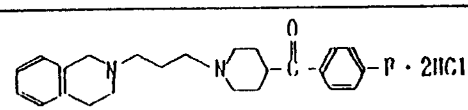 | 199~200 (dec.) | $C_{24}H_{29}N_2OF \cdot 2HCl$ | 63.57<br>63.47 | 6.89<br>6.78 | 6.18<br>6.26 |
| 41 | 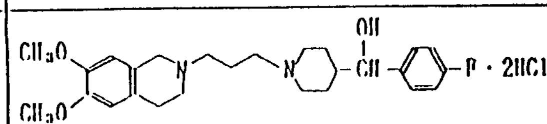 | 198~200 (dec.) | $C_{26}H_{35}N_2O_3F \cdot 2HCl$ | 60.58<br>60.43 | 7.24<br>7.12 | 5.44<br>5.63 |
| 42 | 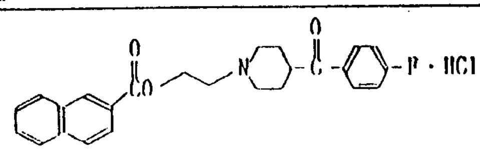 | 209~210 (dec.) | $C_{25}H_{24}NO_3F \cdot HCl$ | 67.94<br>68.01 | 5.70<br>5.81 | 3.17<br>3.01 |
| 43 | 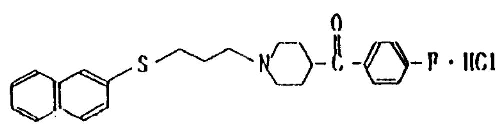 | 195~196 (dec.) | $C_{25}H_{26}NOSF \cdot HCl$ | 67.62<br>67.82 | 6.13<br>6.17 | 3.15<br>2.89 |

Table 1 (cont'd)

| Example No. | Structural formula | Melting point (°C) | Chemical formula | Elementary analysis (%) calculated/found | | |
|---|---|---|---|---|---|---|
| | | | | C | H | N |
| 44 | 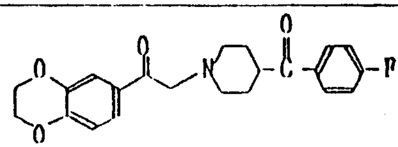 · HCl | 253~254 (dec.) | $C_{22}H_{21}NO_4F \cdot HCl$ | 63.08<br>63.16 | 5.29<br>5.36 | 3.34<br>3.32 |
| 45 | 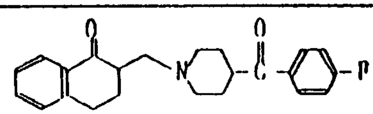 · HCl | 180~181 | $C_{23}H_{24}NO_2F \cdot HCl$ | 68.73<br>68.88 | 6.27<br>6.32 | 3.49<br>3.39 |
| 46 | 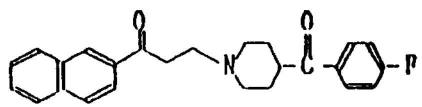 · HCl | 209~210 (dec.) | $C_{25}H_{24}NO_2F \cdot HCl$ | 70.49<br>70.40 | 5.92<br>5.85 | 3.29<br>3.35 |
| 47 | 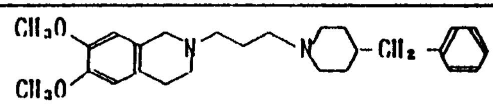 · 2HCl | 266~267 (dec.) | $C_{25}H_{34}N_2O_2 \cdot 2HCl$ | 64.23<br>64.36 | 7.76<br>7.72 | 5.99<br>6.11 |
| 48 | 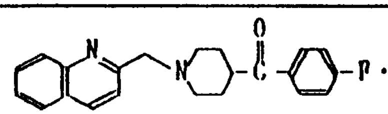 · 2HCl | 214~217 (dec.) | $C_{22}H_{21}N_2OF \cdot 2HCl$ | 62.71<br>62.77 | 5.50<br>5.43 | 6.65<br>6.72 |

Table 1 (cont'd)

| Example No. | Structural formula | Melting point (°C) | Chemical formula | Elementary analysis (%) calculated/found | | |
|---|---|---|---|---|---|---|
| | | | | C | H | N |
| 49 | 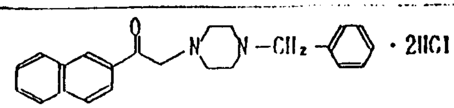 | 260~263 (dec.) | $C_{23}H_{23}N_2OF \cdot 2HCl$ | 63.45<br>63.16 | 5.79<br>5.81 | 6.43<br>6.36 |
| 50 | 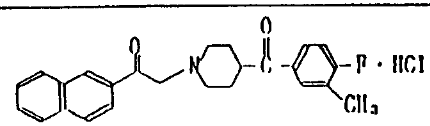 | 236~237 (dec.) | $C_{25}H_{24}NO_2F \cdot HCl$ | 70.50<br>70.31 | 5.92<br>5.86 | 3.29<br>3.31 |
| 51 | 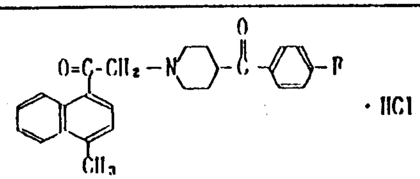 | 242 (dec.) | $C_{25}H_{24}NO_2F \cdot HCl$ | 70.50<br>70.37 | 5.92<br>5.96 | 3.29<br>3.31 |
| 52 | 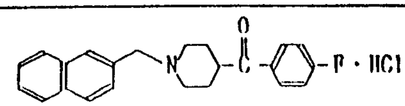 | 237~238 (dec.) | $C_{23}H_{22}NOF \cdot HCl$ | 71.96<br>71.88 | 6.04<br>6.12 | 3.65<br>3.57 |
| 53 | 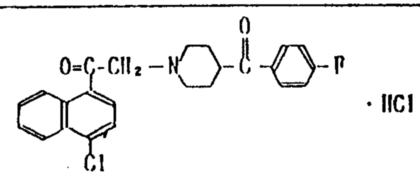 | 231~232 (dec.) | $C_{24}H_{24}NO_2Cl \cdot HCl$ | 69.57<br>69.48 | 6.08<br>6.16 | 3.38<br>3.42 |

Table 1 (cont'd)

| Example No. | Structural formula | Melting point (°C) | Chemical formula | Elementary analysis (%) calculated/found | | |
|---|---|---|---|---|---|---|
| | | | | C | H | N |
| 54 | 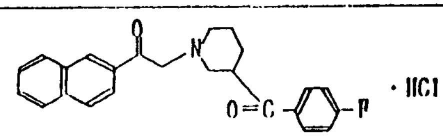 | 153~156 | $C_{24}H_{22}NO_2F \cdot HCl$ | 69.98<br>70.12 | 5.63<br>5.58 | 3.40<br>3.26 |
| 55 | 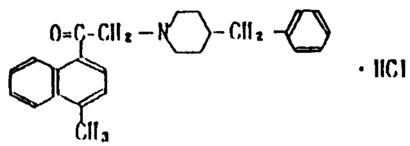 | 222~225 (dec.) | $C_{25}H_{27}NO \cdot HCl$ | 76.22<br>75.93 | 7.16<br>7.33 | 3.56<br>3.47 |
| 56 | 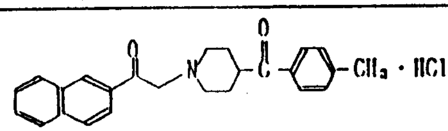 | 250~253 (dec.) | $C_{25}H_{25}NO_2 \cdot HCl$ | 73.61<br>73.48 | 6.42<br>6.33 | 3.43<br>3.19 |
| 57 | 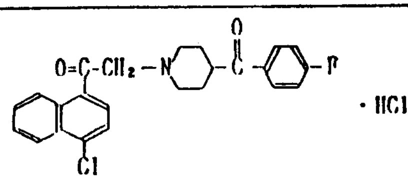 | 256~260 (dec.) | $C_{24}H_{21}NO_2ClF \cdot HCl$ | 67.45<br>67.18 | 5.19<br>5.06 | 3.28<br>3.14 |
| 58 | 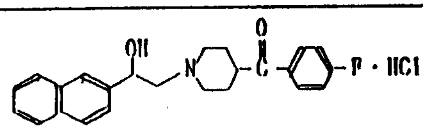 | 246~248 (dec.) | $C_{24}H_{24}NO_2F \cdot HCl$ | 69.64<br>69.61 | 6.08<br>6.02 | 3.38<br>3.14 |

Table 1 (cont'd)

| Example No. | Structural formula | Melting point (°C) | Chemical formula | Elementary analysis (%) calculated/found | | |
|---|---|---|---|---|---|---|
| | | | | C | H | N |
| 59 | 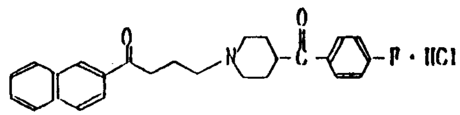 | 250~254 (dec.) | $C_{24}H_{24}NO_2F \cdot HCl$ | 70.98<br>70.96 | 6.19<br>6.14 | 3.18<br>3.20 |
| 60 | 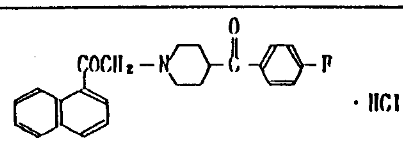 | 223~226 (dec.) | $C_{24}H_{22}NO_2F \cdot HCl$ | 69.98<br>69.86 | 5.63<br>5.58 | 3.40<br>3.22 |
| 61 | 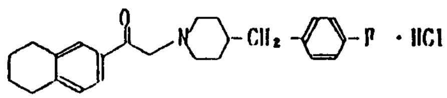 | 272~274 (dec.) | $C_{22}H_{27}NOF \cdot HCl$ | 62.87<br>62.69 | 6.65<br>6.54 | 6.38<br>6.28 |
| 62 | 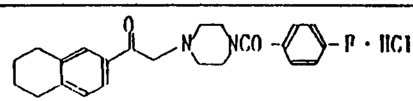 | 214~217 (dec.) | $C_{23}H_{25}N_2O_2F \cdot HCl$ | 66.26<br>66.13 | 6.29<br>6.25 | 6.72<br>6.47 |
| 63 | 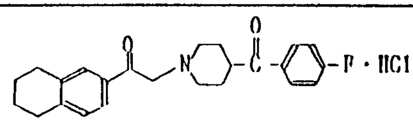 | 263~266 (dec.) | $C_{23}H_{26}NO_2F \cdot HCl$ | 68.39<br>68.18 | 6.74<br>6.55 | 3.47<br>3.41 |
| 64 | 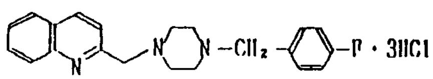 | 234~238 (dec.) | $C_{21}H_{22}N_3F \cdot 3HCl$ | 56.71<br>56.45 | 5.66<br>5.58 | 9.45<br>9.17 |

Table 1 (cont'd)

| Example No. | Structural formula | Melting point (°C) | Chemical formula | Elementary analysis (%) calculated/found C | H | N |
|---|---|---|---|---|---|---|
| 65 | 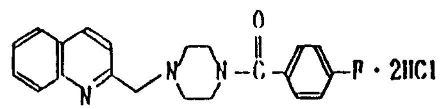 | 230~233 (dec.) | $C_{21}H_{20}N_3OF \cdot 2HCl$ | 59.73<br>59.54 | 5.25<br>5.13 | 9.94<br>9.78 |
| 66 | 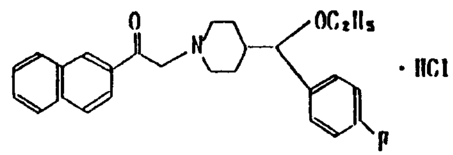 | 142~147 | $C_{26}H_{28}NO_2F \cdot HCl$ | 70.66<br>70.53 | 6.61<br>6.50 | 3.17<br>3.06 |
| 67 | 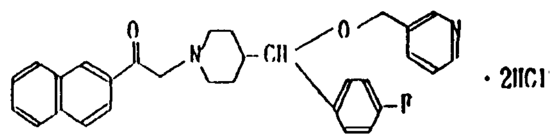 | 98~104 | $C_{30}H_{29}N_2O_2F \cdot 2HCl$ | 66.54<br>66.42 | 5.77<br>5.68 | 5.17<br>5.07 |
| 68 | 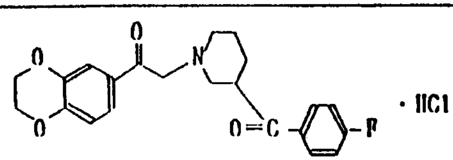 | 135~140 | $C_{22}H_{22}NO_4F \cdot HCl$ | 62.93<br>62.69 | 5.52<br>5.41 | 3.34<br>3.17 |

Table 1 (cont'd)

| Example No. | Structural formula | Melting point (°C) | Chemical formula | Elementary analysis (%) calculated/found | | |
|---|---|---|---|---|---|---|
| | | | | C | H | N |
| 69 | 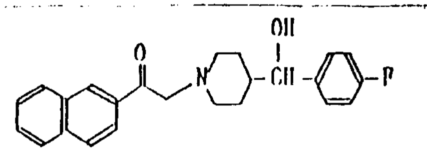 | 162~164 | $C_{25}H_{24}NO_2F$ | 76.37<br>76.03 | 6.41<br>6.40 | 3.71<br>3.56 |
| 70 | 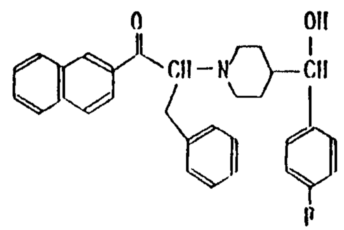 · HCl | 236~237<br>(dec.) | $C_{31}H_{30}NO_2F \cdot HCl$ | 73.86<br>73.92 | 6.20<br>6.21 | 2.78<br>2.78 |
| 71 | 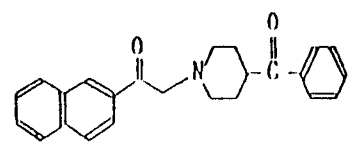 · HCl | 242~245 | $C_{24}H_{23}NO_2 \cdot HCl$ | 73.18<br>73.33 | 6.14<br>6.18 | 3.56<br>3.66 |
| 72 | 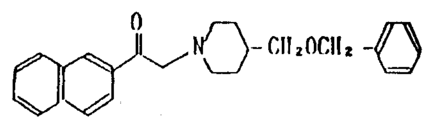 · HCl | 182~183 | $C_{25}H_{27}NO_2 \cdot HCl$ | 73.25<br>73.26 | 6.88<br>6.76 | 3.42<br>3.24 |

Table 1 (cont'd)

| Example No. | Structural formula | Melting point (°C) | Chemical formula | Elementary analysis (%) calculated/found | | |
|---|---|---|---|---|---|---|
| | | | | C | H | N |
| 73 | 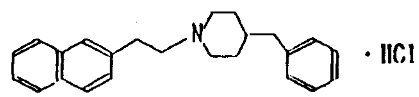 | 222~223 | $C_{24}H_{27}N \cdot HCl$ | 78.77<br>78.73 | 7.11<br>7.64 | 3.83<br>3.62 |
| 74 | 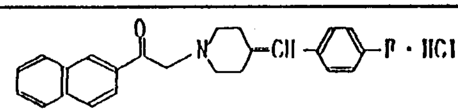 | 246~246.5 | $C_{24}H_{22}NOF \cdot HCl$ | 72.81<br>72.66 | 5.81<br>5.70 | 3.54<br>3.45 |
| 75 | 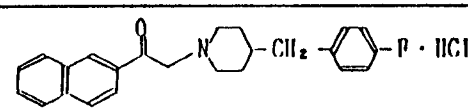 | 243~244 | $C_{24}H_{24}NOF \cdot HCl$ | 72.44<br>72.39 | 6.33<br>6.47 | 3.52<br>3.50 |
| 76 | 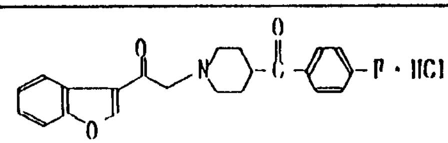 | 224~225 | $C_{22}H_{20}NO_3F \cdot HCl$ | 65.75<br>65.79 | 5.27<br>5.26 | 3.49<br>3.36 |
| 77 | 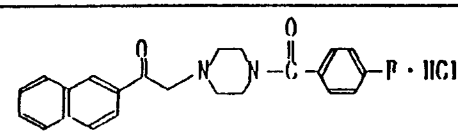 | 206~207 | $C_{23}H_{21}N_2O_2F \cdot HCl$ | 66.90<br>66.82 | 5.37<br>5.36 | 6.78<br>6.80 |

Table 1 (cont'd)

| Example No. | Structural formula | Melting point (°C) | Chemical formula | Elementary analysis (%) calculated/found | | |
|---|---|---|---|---|---|---|
| | | | | C | H | N |
| 78 | 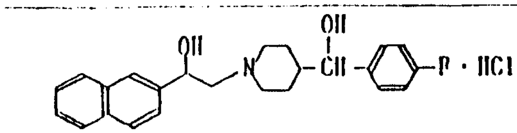 · HCl | 173~174 | $C_{24}H_{27}NO_2F \cdot HCl$ | 69.14<br>69.02 | 6.77<br>6.51 | 3.36<br>3.27 |
| 79 | 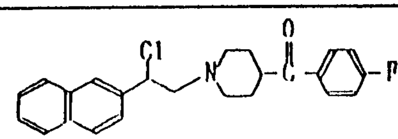 · HCl | 187~188 | $C_{24}H_{23}NOFCl \cdot HCl$ | 66.67<br>66.43 | 5.59<br>5.33 | 3.24<br>3.12 |
| 80 | 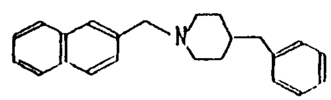 · HCl | 172~173 | $C_{23}H_{25}N \cdot HCl$ | 78.50<br>78.64 | 7.45<br>7.37 | 3.98<br>3.84 |
| 81 | 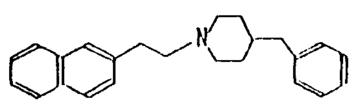 · HCl | 226~227 | $C_{24}H_{27}N \cdot HCl$ | 78.76<br>78.75 | 7.45<br>7.40 | 3.83<br>3.78 |
| 82 | 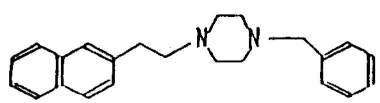 · 2HCl | 274~275 | $C_{23}H_{24}N_2 \cdot 2HCl$ | 68.48<br>68.52 | 7.00<br>7.02 | 6.94<br>6.87 |

Table 1 (cont'd)

| Example No. | Structural formula | Melting point (°C) | Chemical formula | Elementary analysis (%) calculated/found | | |
|---|---|---|---|---|---|---|
| | | | | C | H | N |
| 83 | 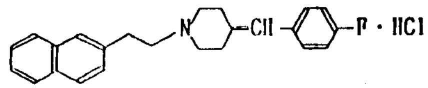 | 249 | $C_{24}H_{24}NF \cdot HCl$ | 75.48<br>75.44 | 6.60<br>6.45 | 3.67<br>3.82 |
| 84 | 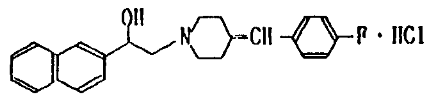 | 203 | $C_{24}H_{24}NOF \cdot HCl$ | 72.44<br>72.11 | 6.33<br>6.19 | 3.52<br>3.59 |
| 85 | 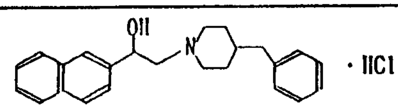 | 216~217 | $C_{24}H_{27}NO \cdot HCl$ | 75.47<br>75.48 | 7.39<br>7.40 | 3.67<br>3.64 |
| 86 | 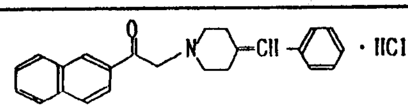 | 239~241 | $C_{24}H_{23}NO \cdot HCl$ | 76.28<br>76.08 | 6.40<br>6.30 | 3.71<br>3.69 |
| 87 | 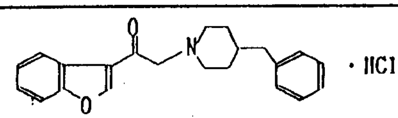 | 221~223 | $C_{22}H_{23}NO_2 \cdot HCl$ | 71.44<br>71.16 | 6.54<br>6.58 | 3.79<br>4.03 |
| 88 | 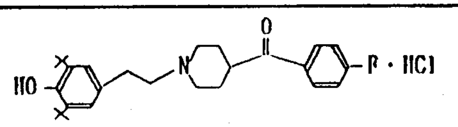 | 227~229 | $C_{28}H_{38}NO_2F \cdot HCl$ | 70.64<br>70.58 | 8.26<br>8.14 | 2.94<br>2.73 |

Table 1 (cont'd)

| Example No. | Structural formula | Melting point (°C) | Chemical formula | Elementary analysis (%) calculated/found C | H | N |
|---|---|---|---|---|---|---|
| 89 | 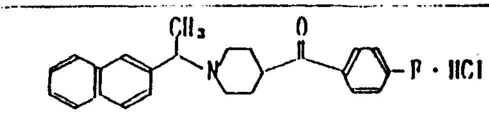 | 205~210 (dec.) | $C_{24}H_{24}NOF \cdot HCl$ | 72.44<br>72.41 | 6.33<br>6.19 | 3.52<br>3.41 |
| 90 | 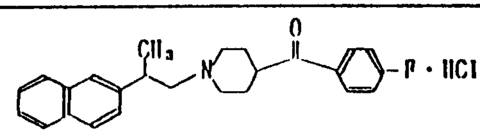 | 195~197 (dec.) | $C_{25}H_{26}NOF \cdot HCl$ | 72.89<br>72.83 | 6.60<br>6.54 | 3.40<br>3.37 |
| 91 | 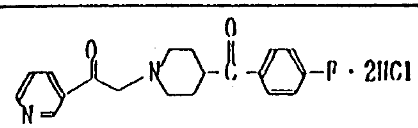 | oily | $C_{19}H_{19}N_2O_2F \cdot 2HCl$ | 57.15<br>56.78 | 5.30<br>5.14 | 7.02<br>6.87 |
| 92 | 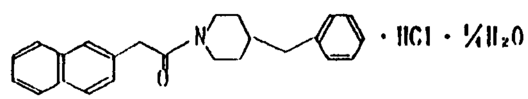 | 233.5~235 | $C_{24}H_{25}NO \cdot HCl \cdot 1/4H_2O$ | 74.98<br>74.90 | 6.95<br>6.92 | 3.64<br>3.69 |
| 93 | 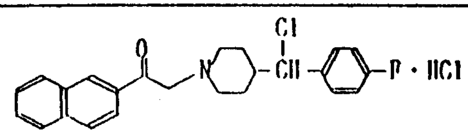 | oily | $C_{24}H_{23}NOFCl \cdot HCl$ | 66.67<br>66.39 | 5.59<br>5.62 | 3.24<br>3.24 |

The examples of pharmacological experiments of the compounds of the present invention will be given below:

Experimental Example 1

Effect of protecting ischemic brain

Carotid arteries of both sides of ICR mice (6 to 8 weeks old) were exposed under halothane anesthesia and ligated. The mice thus treated had stroke symptoms such as jumping, rolling and convulsion and almost all of them died within 24 h.

The compound of the present invention was administered orally to the mice one hour before the ligation and the survival time (maximum: 6 h) was examined as an index of the effect of protecting eschemic brain. In this experiment, the compound was used in the form of a 5% suspension in acacia and a 5% acacia solution was given to the control group.

The results are shown in Table 2. It is apparent that the compounds of the present invention had a life-prolonging effect, while the average survival time of the control group was 149.9 min.

Table 2

| Effect of protecting ischemic brain | | | | |
|---|---|---|---|---|
| Compound used | Dose (mg/kg, p.o.) | Number of cases | Average survival time (min) (average ± S.E.) | % |
| Control group | - | 26 | 149.9 ± 25.8 | 100 |
| Compound of Example 12 | 3 | 10 | 213.7 ± 52.3 | 143 |
| | 10 | 10 | 181.4 ± 43.6 | 121 |
| | 30 | 9 | 191.1 ± 54.3 | 128 |
| Compound of Example 73 | 10 | 7 | 150.4 ± 57.6 | 100 |
| | 30 | 6 | 275.2 ± 58.2 | 184 |
| Compound of Example 74 | 3 | 10 | 143.3 ± 39.6 | 96 |
| | 10 | 7 | 205.1 ± 43.6 | 137 |
| | 30 | 7 | 194.2 ± 49.7 | 130 |

Experimental Example 2

Effect of remedying learning disorder after ischemia

Common carotid arteries on both sides of Mongolian gerbils (17 to 21 weeks old) were clipped with Skoville clamps without anesthesia and the clamps were removed after 5 min to realize a short period of ischemia. Twenty-four hours after the removal of the clamps, these animals were subjected to learning and memory tests were conducted after additional 24 h.

The learning and memory functions were examined by the passive avoidance method with a modification of a device reported by Jarvik & Kopp in "Psychological Reports", 21, 221 to 224 (1967). The device had two chambers, i.e. a well-lighted chamber A and a dark chamber B. In the tests, the animals were placed in the well-lighted chamber A and an electric current (A.C., 1.6 mA) was applied to a grid on the floor of the dark chamber B for 5 min when they entered the chamber B.

On the next day, the animals subjected to the learning were placed in the chamber A and the time (latent time) which had elapsed before they entered the chamber B was measured. The upper limit of the latent time was set at 300 sec.

The compound was administered in the form of a 5% suspension in acacia orally one hour before causing the ischemia. A 5% acacia solution was administered to the control group.

The results are shown in Table 3. The average latent time of the normal (pseudo-operation) group was 246.5 sec and that of the control group was as short as 71.5 sec. Namely, the learning and memory functions of them were damaged by the 5-min ischemia. When the compounds of the present invention were administered to the control group, the latent time was elongated again, namely the learning disorder

after the ischemia was remedied.

Table 3 Effects of remedying learning disorder after ischemia

| Compound used | Dose (mg/kg, p.o.) | Number of cases | Latent time (sec) (average ± S.E.) | Recovery ratio (%) * |
|---|---|---|---|---|
| Normal group | — | 65 | 246.5 ± 10.9 | 100 |
| Control group | — | 62 | 71.5 ± 11.7 | 0 |
| Compound of Example 12 | 3 | 22 | 168.8 ± 23.0 | 56 |
| | 10 | 24 | 196.8 ± 22.3 | 72 |
| | 30 | 11 | 196.3 ± 37.0 | 71 |
| Compound of Example 73 | 10 | 8 | 193.1 ± 35.3 | 69 |
| | 30 | 7 | 80.1 ± 28.2 | 5 |
| Compound of Example 74 | 3 | 13 | 110.2 ± 29.0 | 22 |
| | 10 | 24 | 123.2 ± 24.3 | 30 |
| | 30 | 21 | 129.2 ± 23.8 | 33 |

* The recovery ratio was calculated according to the following formula for each latent time:

$$\frac{\text{(treated group)} - \text{(control group)}}{\text{(normal group)} - \text{(control group)}} \times 100$$

Experimental Example 3

Effect of protecting cells from disorder after ischemia

Carotid arteries on both sides of Mongolian gerbils were blocked to realize cerebral ischemia for 5 min. As a result, the nerve cells in the CAI region of the hippocampus disappeared extensively [Karino, T.: Brain Res., 239, 57 to 69 (1982)].

The compound of the present invention was administered orally to them, while a 5% acacia suspension was administered to the control group. After one hour, the ischemia was realized for 5 min. After one week, the animal was perfused and fixed with 4% neutral formalin transcardially. The treated sample was

embedded in paraffin and cut to obtain slices having a thickness of 3 µm. The slices were dyed with hematoxylin-eosin and the number of the nerve cells in the CAI region of the hippocampus of each slice was counted.

The results are shown in Table 4. The nerve cell density in the CAI region of the hippocampus was 287/mm in the normal (pseudo-operation) group and that of the control group was as small as 21/mm. Namely, a serious disappearance of the cell was caused by the 5-min ischemia. On the other hand, when the compound of the present invention was administered, the nerve cell density was increased to prove the effect thereof in protecting the cells from the disorder.

Table 4

| Effect of protecting the cells from disorder after ischemia | | | |
|---|---|---|---|
| Compound used | Dose (mg/kg, p.o.) | Number of cases | Nerve cell density (number/mm) |
| Normal group | - | 6 | 287 ± 6 |
| Control group | - | 16 | 21 ± 10 |
| Compound of Example 12 | 3 | 8 | 62 ± 26 |
| | 10 | 10 | 75 ± 32 |
| | 30 | 10 | 83 ± 32 |
| Compound of Example 73 | 10 | 7 | 69 ± 21 |
| | 30 | 5 | 49 ± 8 |
| Compound of Example 74 | 30 | 8 | 62 ± 5 |

## Claims

**1.** Cyclic amine derivatives of the general formula or pharmacologically acceptable salt thereof:

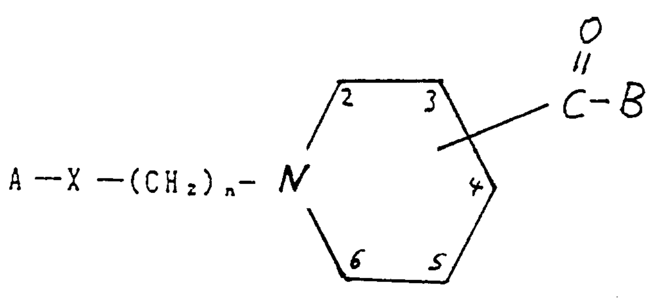

wherein A is naphthyl or a naphthyl having a substituent selected from a halogen and a $C_1$ to $C_6$ alkyl;
X is is -CO or -CH(OH)-;
n is 0 to 4 and
B is phenyl or a phenyl having a substituent selected from a halogen and a $C_1$ to $C_6$ alkyl.

**2.** A cyclic amine derivative or a pharmacologically acceptable salt thereof according to claim 1 of the formula

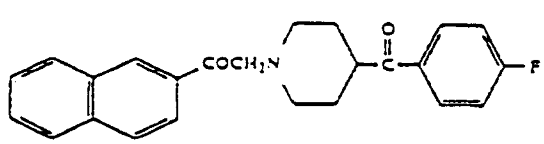

which is 2-[4-(p-fluorobenzoyl)-1-piperidinyl]-2-acetonaphthone.

**3.** A cyclic amine derivative or a pharmaceutically acceptable salt of a compound of the formula:

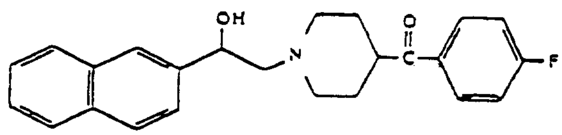

which is
2-[4-p-fluorobenzoyl)-1-piperidinyl]-1-naphthylethanol.

**4.** A process for producing a cyclic amine derivative according to the formula in claim 1, characterized by reacting a halide of the general formula

$$A-X-(CH_2)_{n-1}-CH_2-Hal$$

wherein Hal represents a halogen atom and A, X and n are as defined before with a comound of the general formula

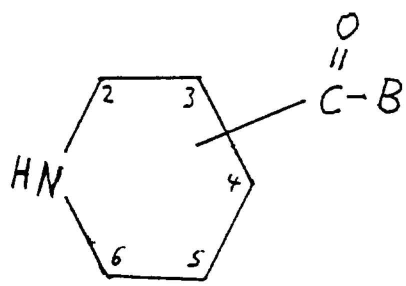

wherein B is as defined before to form a cyclic amine derivative of the general formula of claim 1 and, if necessary, converting this compound into a pharmacologically acceptable salt thereof.

**5.** A medicine for relieving, curing or preventing mental symptoms due to cerebral vascular disorders, which contains as active ingredient a cyclic amine derivative of the general formula of claim 1 or a pharmacologically acceptable salt thereof.

**6.** Use of a compound of the general formula of claim 1 or a pharmacologically acceptable salt thereof for preparing a medicament for relieving, curing or preventing mental symptons due to cerebral vascular disorders.

## Patentansprüche

**1.** Cyclische Aminderivate der allgemeinen Formel

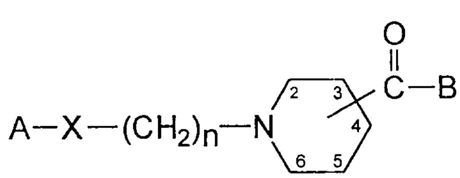

oder deren pharmakologisch annehmbare Salze, worin
A Naphthyl oder eine Naphthylgruppe mit einem Substituenten, ausgewählt aus Halogen und $C_{1-6}$-Alkyl ist;
X -CO oder -CH(OH)- ist
n 0 bis 4 ist und
B Phenyl oder eine Phenylgruppe mit einem Substituenten, ausgewählt aus Halogen und $C_{1-6}$-Alkyl, ist.

2. Cyclisches Aminderivat oder eines seiner pharmakologisch annehmbaren Salze gemäss Anspruch 1 der Formel

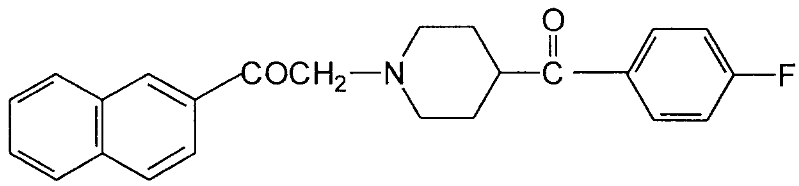

welche 2-[4-(p-Fluorbenzoyl)-1-piperidinyl]-2-acetonaphthon ist.

3. Cyclisches Aminderivat oder ein pharmazeutisch annehmbares Salz einer Verbindung der Formel

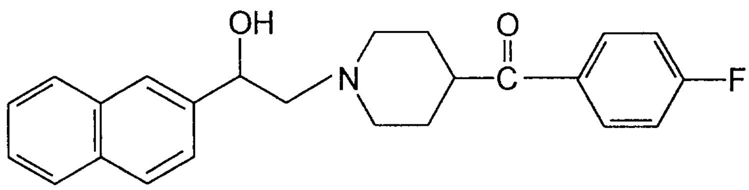

welche 2-[4-(p-Fluorbenzoyl)-1-piperidinyl]-1-naphthylethanol ist.

4. Verfahren zur Herstellung eines cyclischen Aminderivats gemäss der Formel in Anspruch 1, **gekennzeichnet** durch Umsetzen eines Halogenids der allgemeinen Formel

$$A-X-(CH_2)_{n-1}-CH_2-Hal$$

worin Hal ein Halogenatom darstellt und A, X und n wie zuvor definiert sind, mit einer Verbindung der allgemeinen Formel

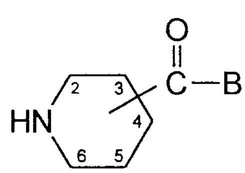

worin B wie zuvor definiert ist, zur Bildung eines cyclischen Aminderivats der allgemeinen Formel des Anspruchs 1 und, falls nötig, Umwandlung dieser Verbindung in eines ihrer pharmakologisch annehmbaren Salze.

5. Arzneimittel zur Linderung, Heilung oder Verhinderung von mentalen Symptomen aufgrund von cerebralen Gefässstörungen, das als aktiven Bestandteil ein cyclisches Aminderivat der allgemeinen Formel des Anspruchs 1 oder eines seiner pharmakologisch annehmbaren Salze enthält.

6. Verwendung einer Verbindung der allgemeinen Formel des Anspruchs 1 oder eines seiner pharmakologisch annehmbaren Salze zur Herstellung eines Medikaments zur Linderung, Heilung oder Verhinderung von mentalen Symptomen aufgrund von cerebralen Gefässstörungen.

## Revendications

1. Dérivés d'amines cycliques, ou sels pharmacologiquement acceptables de ceux-ci, de formule générale :

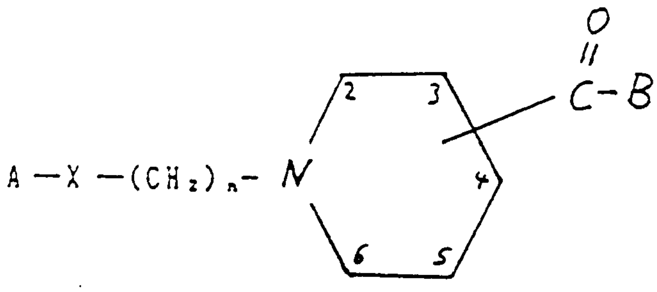

dans laquelle A est un groupe naphtyle ou naphtyle ayant un substituant choisi parmi un halogène et un groupe alkyle en $C_1$ à $C_6$ ;
X est -CO- ou -CH(OH)- ;
n vaut 0 à 4 et
B est un groupe phényle ou phényle ayant un substituant choisi parmi un halogène et un groupe alkyle en $C_1$ à $C_6$.

2. Dérivé d'amine cyclique ou sel pharmacologiquement acceptable de celui-ci selon la revendication 1 répondant à la formule

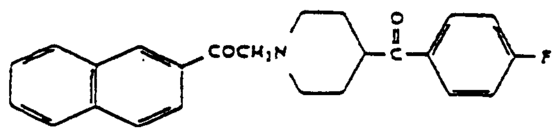

qui est la 2-[4-(p-fluorobenzoyl)-1-pipéridinyl)-2-acétonaphtone.

3. Dérivé d'amine cyclique ou sel pharmaceutiquement acceptable d'un composé de formule :

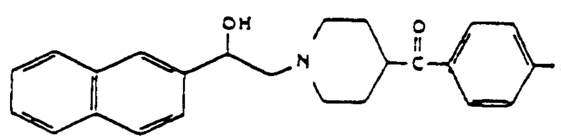

qui est le 2-[4-(p-fluorobenzoyl)-1-pipéridinyl]-1-naphtyléthanol.

4. Procédé pour préparer un dérivé d'amine cyclique répondant à la formule de la revendication 1, caractérisé par la réaction d'un halogénure de formule générale

$$A-X-(CH_2)_{n-1}-CH_2-Hal$$

dans laquelle Hal représente un atome d'halogène et A, X et n sont définis comme précédemment, avec un composé de formule générale

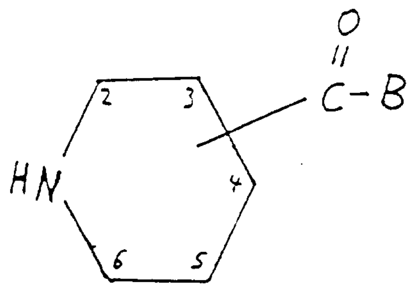

dans laquelle B est défini comme précédemment, pour former un dérivé d'amine cyclique répondant à la formule générale de la revendication 1 et, si nécessaire, la conversion de ce composé en un de ses sels pharmacologiquement acceptables.

**5.** Médicament pour le soulagement, la guérison ou la prévention des symptômes mentaux dus aux troubles cérébro-vasculaires, qui contient comme ingrédient actif un dérivé d'amine cyclique répondant à la formule générale de la revendication 1 ou un sel pharmacologiquement acceptable de celui-ci.

**6.** Utilisation d'un composé répondant à la formule générale de la revendication 1 ou d'un sel pharmacologiquement acceptable de celui-ci pour la préparation d'un médicament pour le soulagement, la guérison ou la prévention des symptômes mentaux dus à des troubles cérébro-vasculaires.